(19) Europäisches Patentamt
European Patent Office
Office européen des brevets

(11) Publication number : **0 442 585 B1**

(12) **EUROPEAN PATENT SPECIFICATION**

(45) Date of publication of patent specification : **20.07.94 Bulletin 94/29**

(51) Int. Cl.$^5$ : **C07C 237/20,** C07C 231/20, C07B 55/00

(21) Application number : **91200307.6**

(22) Date of filing : **14.02.91**

# (54) Process for racemization of an optically active amino acid amide.

(30) Priority : **16.02.90 NL 9000387**

(43) Date of publication of application : **21.08.91 Bulletin 91/34**

(45) Publication of the grant of the patent : **20.07.94 Bulletin 94/29**

(84) Designated Contracting States : **AT BE CH DE DK ES FR GB GR IT LI NL SE**

(56) References cited :
**EP-A- 0 057 092**
**EP-A- 0 199 407**
**FR-A- 2 334 659**
**US-A- 4 072 698**

(73) Proprietor : **DSM N.V.**
**Het Overloon 1**
**NL-6411 TE Heerlen (NL)**

(72) Inventor : **Boesten, Wilhelmus Hubertus Joseph**
**Brountslaan 9**
**NL-6132 BJ Sittard (NL)**

**EP 0 442 585 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the racemization of an optically active amino acid amide by reacting the amino acid amide with a carboxylic acid in the presence of a solvent and an aldehyde.

In US-A-4072698 a process is described in which optically pure nitriles, amides or Schiff bases of nitriles or amides are prepared with an optically active acid in the presence of a ketone or an aldehyde. That publication is notably aimed at the preparation of optically pure nitriles, in particular 2-amino-2- (optionally p-substituted) phenyl acetonitrile, using optically pure tartaric acid and a ketone. In such a process, racemization of the undesired enantiomer takes place implicitly.

The yield of salt of nitrile and tartaric acid in the examples described in said patent specification is relatively low, however, i.e. not higher than 85%. In respect of amino acid amides and Schiff bases, no experimental data are mentioned, nor are claims made for the process with such compounds as starting material.

In EP-A-0 057 092 the racemization of an optically active aminoacid in the presence of an aliphatic acid and an aldehyde is disclosed.

In EP-A-0 199 407 the racemization of the Schiff base of an optically active aminoacid amide in the presence of a strong base is disclosed.

The invention now aims to provide a process with which rapid racemization of optically active amino acid amides is effected without significant losses occurring and with which a high yield of amino acid amide or salt of amino acid amide and carboxylic acid can be obtained.

According to the invention this is due to the addition of water to the reaction mixture and the quantity of aldehyde amounting to 0.5-4 equivalents relative to the quantity of amino acid amide.

For is has appeared that also if an amino acid amide and an aldehyde are started from, poor racemization degrees are obtained if an insufficient quantity of aldehyde is used or that if no water, that is to say, for instance less than 0.01 equivalent relative to the amide, is added to the reaction mixture, the yield obtained is low. Although the invention is not bound to any theoretical explanation, it seems that due to the presence of water, fewer side reactions occur, so that the losses are lower. In practice mostly 0.1-4 equivalents of water relative to the amide are used, preferably 0.5-3 equivalents.

Surprisingly it has appeared that the racemization proceeds with a high yield and a good degree of racemization if water is present in the reaction medium. This is so surprising because the racemization proceeds via the formation and racemization of the Schiff base, the formation of which is inhibited by the presence of water. After all, it is to be expected that the presence of water has a negative effect on the establishment of equilibrium in the formation of the Schiff base, which is expected to delay the occurrence of racemization. Moreover, water enhances the solubility of the diastereoisomeric salt which is formed as an intermediate, so that less salt is recovered in solid form, which implies that a lower yield should be expected.

Racemization occurs for instance in asymmetric transformations as described in 'Enantiomers, Racemates and Resolutions', Jean Jacques, André Collet, Samuel H. Wilen; John Wiley & Sons, New York (1981), pp. 369 ff.

Aldehydes used in the racemization are for instance aromatic aldehydes, such as benzaldehyde, anisaldehyde, o-, p-, or m-nitrobenzaldehyde, o-, p- or m-chlorobenzaldehyde or aliphatic aldehydes, such as isobutyraldehyde or isovaleraldehyde. The quantity of aldehyde to be added is 0.5-4.0 equivalents relative to the quantity of amino acid amide, preferably 1-2 equivalents.

Instead of an optically active amino acid amide and an aldehyde, the starting material used for the preparation of a racemic mixture of L- and D-amino acid amide can also be the corresponding optically active Schiff base. In that case it is not necessary to use an extra quantity of aldehyde. In order to obtain an optimum yield of the salt of amino acid amide and carboxylic acid, at least an equimolar quantity of water relative to the quantity of Schiff base should be added.

The quantity of water preferably used in the process according to the invention varies according to the amino acid amide chosen, and can be easily determined by one skilled in the art. In general this quantity will be between 0.1 and 4 equivalents relative to the quantity of amino acid amide, preferably between 0.5 and 3 equivalents.

It is of no importance whether the water is added at the beginning of the reaction or in the course of the conversion process. The water can be added in any desired manner, for instance as diluent of the reactants or the solvent. Preferably, a sufficient quantity of water is added previously.

From US-A-4094904 a process is known for the racemization of optically active phenylglycine amide, in which an optically active phenylglycine amide is treated with an acid in the presence of ketones. The reaction time this process requires is much longer, however, than that of the process according to the invention.

In the process according to the invention carboxylic acids are used. The acid strength (pKa) will in general be 3 to 5. Examples of suitable carboxylic acids are acetic acid, mandelic acid, propionic acid, benzoic acid

and 2-pyrrolidone-5-carboxylic acid.

The quantity of carboxylic acid to be used can be varied within wide limits. Preferably an excess of carboxylic acid is used.

Suitable solvents for the racemization are for instance hydrocarbons, such as cyclohexane, heptane and octane, aromatic hydrocarbons, such as toluene, xylene and benzene, ethers, such as methyl tertiary butyl ether, dioxane, tetrahydrofuran and anisole, esters, such as butyl acetate and ethyl acetate, ketones, such as acetone, butanone, methyl isobutyl ketone, carboxylic acids, aldehydes or mixtures of these substances. It will be clear that a solvent should be chosen which does not enter into irreversible chemical reactions with the amino acid amide, the carboxylic acid or the aldehyde.

The pressure at which the process according to the invention is carried out is not critical. The process is preferably carried out at atmospheric pressure. The temperature can be varied within wide limits and is in general 20-120°C, preferably 75-100°C. The reaction time is mostly 1-24 hours, depending on, among other factors, the amino acid amide and the temperature chosen, but is preferably 1-4 hours.

The slurry concentration of the salts is about 5-30 wt.%, preferably 10-20 wt.%.

The invention will now be elucidated by means of the examples, without being restricted thereto. Each experiment is carried out in a nitrogen atmosphere.

The analysis method used is thin-layer chromatography (TLC), with:
Merck 60 F 254 silicagel being used as carrier; UV (short wave) and ninidrine being used as detection methods.

## Example I

In a flask provided with a stirrer, a thermometer and a reflux cooler, 23.8 g (0.10 mole) D-N-benzylidene phenylglycine amide (selectivity: 99.5%), 15.2 g (0.10 mole) D,L-mandelic acid, 200 ml toluene, 50 ml ethyl acetate and 2.7 ml (0.15 mole) water are stirred for 4 hours at a temperature of 85°C.
After cooling to 25°C the mandelate of D,L-phenylglycine amide is filtered and washed on the glass filter with 4 x 25 ml toluene. The yield of TLC pure mandelate of D,L-phenylglycine amide amounts to 29.9 g, which corresponds to an efficiency of 99.1%.
1.0 g of the mandelate of D,L-phenylglycine amide is added to a mixture of 10 ml water and 10 ml 12 N hydrochloric acid. The D,L-phenylglycine amide.HCl formed is filtered and washed on the glass filter with 4 x 5 ml acetone. The yield of TLC pure D,L-phenylglycine amide.HCl amounts to 0.57 g (efficiency = 92.3%). The specific rotation of the D,L-phenylglycine amide.HCl is:

$$[\alpha]^{20}_{D} = -0.6° \ (c = 0.8; \text{water})$$

The selectivity is: 50.3% D-enantiomer.

## Comparative experiment A

Example I is repeated, but without addition of water. The mandelate of D,L-phenylglycine amide is now obtained with an efficiency of 40.4% (12.2 g). The specific rotation of the D,L-phenylglycine amide.HCl obtained is now:

$$[\alpha]^{20}_{D} = -0.3° \ (c = 0.8; \text{water}).$$

The selectivity is: 50.1% D-enantiomer.

## Examples II-VIII

Example I is repeated for different amides or Schiff bases (SB) thereof. As solvent a mixture of toluene (t) and ethyl acetate (e) is used, the volume ratio (t/e) of which is shown in table 1. Benzaldehyde is used as aldehyde. The starting products, the substances added and their quantities, as well as the results are shown in table 1. The three TLC eluents and the proportions by volume in which they are used are:

| | | | |
|---|---|---|---|
| A | $CHCl_3$ - | $CH_3OH$ - | $NH_4OH$ (25 wt.%) |
| | 60 | 45 | 20 |

| | | | | |
|---|---|---|---|---|
| B | sec. butanol | - formic acid | - water | |
| | 75 | 15 | 10 | |
| C | n-butanol | - acetic acid | - ethyl acetate | - water |
| | 1 | 1 | 1 | 1 |

The selectivity (enantiomeric purity) is defined as follows:

$$\text{selectivity} = 50\% + \frac{50 \times [\alpha]^{20}_D}{\text{max. } [\alpha]^{20}_D}\%$$

The maximum specific rotations of a number of amino acid amides and/or their salts are given in Greenstein and Winitz, vol. 2, pp. 1196-2000, as well as in Beilstein 14 III, p. 1189.
The maximum specific rotations of some amino acid amides and/or their salts are also given in US-A-4 847 412: D-phenylglycine amide.HCl: -100.8° (c = 0.8; water) D-methionine amide.HCl: -18.2° (c = 1.0; water) D-homophenylalanine amide.1/2 $H_2SO_4$: -15.7° (c = 1.0; water) L-phenylalanine amide.1/2 $H_2SO_4$: +17.8° (c = 1.0; water) From the applicant's own observations the maximum specific rotation of D-p-hydroxyphenylglycine amide is known: -121.5° (c = 1.0; 1.0 N acetic acid).

Table 1

| ex. | solvent | starting product | water (eq.) | aldehyde (eq.) | slurry conc. | T | time | effic. | select. |
|---|---|---|---|---|---|---|---|---|---|
| II | t/e = 4/1 | SB, L-valine amide | 1.5 | 0 | 10% | $85^oC$ | 3h | 96.6% | 96.1% |
| VI | t/e = 4/1 | SB, D-leucine amide | 1.5 | 0 | 11% | $85^oC$ | 3h | 95.4% | 85.8% |
| IV | t/e = 4/1 | SB, D-homophenylalanine amide | 1.5 | 0 | 13% | $85^oC$ | 3h | 88.2% | 72.9% |
| V | t/e = 3/1 | SB, D-methionine amide | 1.5 | 0 | 7% | $80^oC$ | 3h | 82.0% | 67.6% |
| VI | t/e = 4/1 | SB, D-phenylglycine amide | 1.5 | 0 | 12% | $85^oC$ | 3h | 98.7% | 51.2% |
| VII | t/e = 4/1 | D-p-hydroxyphenylglycine amide | 2.5 | 1 | 13% | $85^oC$ | 3h | 97.5% | 61.7% |
| VIII | t/e = 4/1 | SB, L-phenylalanine amide | 1.5 | 0 | 13% | $85^oC$ | 3h | 92.4% | 59.3% |

Examples IX - XIII and comparative experiment B

In the same manner as in example I, optically active phenylglycine amide or its corresponding base (selectivity at t = 0 : 100%) is racemized in different mixtures of acetic acid, benzaldehyde and water as solvent. The composition of the solvent and the results obtained at given reaction time (t, in minutes) are shown in table 2.

At higher temperatures and with an excess of carboxylic acid the racemization is significantly accelerated. If benzaldehyde is absent, there is no racemization.

Table 2

| example | solvent | starting product | percentage PGA | eq. a.e. | eq. water | eq. BA | T | $[\alpha]_{t=0}$ | t [min.] | $[\alpha]_{t=t}$ | selectivity |
|---|---|---|---|---|---|---|---|---|---|---|---|
| IX | a/w = 99/1 | D-SB-PGA | 4.0% | 95 | 3.0 | 0 | 25°C | -2.661° | 90 | -0.166° | 53.1% |
| B | a/w = 99/1 | D-PGA | 2.5% | 95 | 3.0 | 0 | 25°C | -4.075° | 30 | -4.055° | 99.8% |
| X | BA | D-PGA | 3.6% | 1 | 1.5 | 66.6 | 25°C | -0.555° | 45 | -0.548° | 99.4% |
| XI | BA | D-PGA | 3.6% | 1 | 1.5 | 66.6 | 80°C | -0.555° | 20 | -0.225° | 70.3% |
| XII | BA | D-PGA | 2.8% | 22 | 2.0 | 66.6 | 25°C | -0.375° | 90 | -0.240° | 82.0% |
| | | | | | | | | | 150 | -0.175° | 73.3% |

Abbreviations: a/w = acetic acid/water = acetic acid/water volume proportion in an acetic acid/water mixture

BA = benzaldehyde

a.e. = acetic acid

SB-PGA = Schiff base of phenylglycine amide and benzaldehyde

Table 2 shows that the presence of aldehyde, as such or in the form of the Schiff base, is required for the occurrence of racemization. Further, the effect of the temperature on the rate of racemization is demonstrated.

Examples XIII-XIX

The Schiff base (SB) of different amino acid amides (selectivity at t = 0 : 100%), as indicated in table 3, is racemized as a 2 wt.% solution at a temperature of 25°C in the same manner as in example I. As solvent is used a mixture of acetic acid and water with an acetic acid/water ratio of 99/1).

The angle of rotation $[\alpha]_t$ and the selectivity (sel.) after 30 and after about 1200 minutes are shown in table 3.

Also at this low racemization temperature, racemization occurs relatively fast.

Table 3

| Ex. | SB | $[\alpha]_{t=0}$ | t | $[\alpha]_{t=t}$ | sel. |
|---|---|---|---|---|---|
| XIII | D-leucine amide | -0.525° | 30 | -0.492° | 96.9% |
| | | | 1210 | -0.341° | 82.5% |
| XIV | L-alanine amide | +1.970° | 30 | +1.450° | 86.8% |
| | | | 1200 | +0.066° | 51.7% |
| XV | D-o-chlorophenyl-glycine amide | -0.730° | 30 | -0.500° | 84.2% |
| | | | 1150 | -0.007° | 50.5% |
| XVI | D-homophenyl-alanine amide | -0.095° | 30 | -0.063° | 83.2% |
| | | | 1140 | -0.050° | 76.3% |
| XVII | L-methionine amide | -0.190° | 30 | -0.078° | 70.5% |
| | | | 1100 | 0.000 | 50.0% |
| XVIII | D-phenylalanine amide | +2.160° | 30 | +1.790° | 91.4% |
| | | | 1080 | +0.327° | 57.6% |
| XIX | D-phenylglycine amide | -1.626° | 30 | -1.140° | 85.1% |
| | | | 1070 | 0.000° | 50.0% |

Example XX

Asymmetric transformation of D,L-phenylglycine amide with mandelic acid (in situ racemization).

In a reaction flask provided with a stirrer, a thermometer and a reflux cooler, 15.0 g (0.10 mole) D,L-phenylglycine amide, 15.2 g (0.10 mole) D-mandelic acid, 230 ml of a solvent which is a mixture of ethyl acetate and toluene with a volume proportion of 1 to 3, 20.4 g anisaldehyde (0.15 mole) and 1.8 g (0.10 mole) water are stirred for 3.5 hours at a temperature of 84°C.

After cooling to 20°C (0.5 hour) the resulting diastereoisomeric salt of L-phenylglycine amide and D-mandelic acid (LD salt) is filtered and washed on the glass filter with 4 x 25 ml toluene. The yield of TLC-pure LD salt after drying amounts to 29.2 g, which corresponds to an efficiency of 96.7%.

The specific rotation of the optically pure LD salt (recrystallization) is:

$$[\alpha]^{20}_D = +4.0° \text{ (c = 1.0; water)}.$$

Of the diastereoisomeric LD salt obtained, 1.0 g is suspended in 10 ml water, after which 10 ml 12 N hydrochloric acid is added with stirring. After filtration and washing with 4 x 10 ml acetone on the glass filter of the L-phenylglycine amide.HCl crystal mass obtained, the specific rotation of the resulting TLC-pure L-phenylglycine amide.HCl salt (yield = 0.55 g; efficiency = 88.7%) is:

$[\alpha]^{20}{}_D$ = + 102.1° (c = 0.8; water).

The selectivity, i.e. the optical purity, of the phenylglycine amide.HCL salt is 99.8%.

Example XXI

In the same manner as in example XX, 2 eq. isobutyraldehyde is added, the solvent used being an ethyl acetate/toluene mixture with a mixing ratio of 1/4. Stirring is continued for 3 hours at 80°C. The efficiency is now 92.4%; the selectivity is 99.8%.

Example XXII

Asymmetric transformation of D,L-p-hydroxyphenylglycine amide with L-mandelic acid (in situ racemization).

In a reaction flask provided with a stirrer, a thermometer and a reflux cooler, 8.3 g (0.05 mole) D,L-p-hydroxyphenylglycine amide, 7.6 g (0.05 mole) L-mandelic acid, 125 ml toluene, 25 ml dioxane, 14.1 g (0.1 mole) o-chlorobenzaldehyde and 0.9 g (0.05 mole) water are stirred for 2.5 hours at a temperature of 86°C.

After cooling to 30°C (3/4 hour) the resulting diastereoisomeric salt of D-p-hydroxyphenylglycine amide and L-mandelic acid (DL salt) is filtered and washed on the glass filter with 5 x 20 ml ethyl acetate. The yield of TLC-pure DL salt amounts to 14.4 g, which corresponds to an efficiency of 90.3%. The specific rotation of the optically pure DL salt (recrystallization) is:

$[\alpha]^{20}_D$ = - 8.1° (c = 1.0; water).

1.6 g of the DL salt is dissolved in 20 ml water at 50°C, after which 1 ml 25 wt.% ammonia is added with stirring. After cooling to 20°C, filtration and washing of the resulting D-p-hydroxyphenylglycine amide crystals with 3 x 10 ml water and 3 x 10 ml methanol, respectively, the specific rotation of the TLC-pure D-p-hydroxyphenylglycine amide (yield = 0.8 g; efficiency = 95.8%) is:

$[\alpha]^{20}_D$ = - 121° (c = 1.0; 1.0 N acetic acid).

The percentage of D-enantiomer is 99.8.

## Claims

1. Process for the racemization of an optically active amino acid amide by reacting the amino acid amide with a carboxylic acid in the presence of a solvent and an aldehyde, characterized in that water is added to the reaction mixture and that the quantity of aldehyde amounts to 0.5-4 equivalents relative to the quantity of amino acid amide.

2. Process according to claim 1, characterized in that the quantity of water is 0.5-3 equivalents with respect to the amide.

3. Process according to claim 1-2, characterized in that the quantity of aldehyde amounts to 1-2 equivalents calculated relative to the quantity of amino acid amide.

4. Process for the racemization of an optically active Schiff base of an amino acid amide by reacting the Schiff base with a carboxylic acid in the presence of a solvent, characterized in that before and/or during the reaction a quantity of water which is at least equivalent to the quantity of Schiff base is added as well to the reaction mixture.

5. Process according to any one of the claims 1-4, the water being added at the beginning of the reaction.

6. Process according to any one of the claims 1-5, characterized in that the reaction mixture is kept at a temperature between 75 and 100°C.

7. Process according to any one of the claims 1-6, the amino acid amide being phenylglycine amide.

8. Process according to any one of the claims 1-6, characterized in that the amino acid amide is alanine amide.

9. Process according to any one of the claims 1-6, characterized in that the amino acid amide is metionine

amide.

10. Process according to any one of the claims 1-6, characterized in that the amino acid amide is o-chlorophenylglycine amide.

11. Process according to any one of the claims 1-10, characterized in that subsequently the resulting salt of the racemized amino acid amide and the carboxylic acid is recovered.

## Patentansprüche

1. Verfahren zur Racemisierung eines optisch aktiven Aminosäureamids durch Umsetzen des Aminosäureamids mit einer Carbonsäure in Gegenwart eines Lösungsmittels und eines Aldehyds, dadurch gekennzeichnet, daß Wasser dem Reaktionsgemisch zugegeben wird und daß die Menge an Aldehyd 0,5-4 Äquivalente, relativ zur Menge an Aminosäureamid, ausmacht.

2. Verfahren nach Anspruch 1, dadurch gekennzeichnet, daß die Menge an Wasser 0,5-3 Äquivalente hinsichtlich des Amids ist.

3. Verfahren nach Anspruch 1-2, dadurch gekennzeichnet, daß die Menge an Aldehyd 1-2 Äquivalente, berechnet relativ zur Menge an Aminosäureamid, ausmacht.

4. Verfahren zur Racemisierung einer optisch aktiven Schiffschen Base aus einem Aminosäureamid durch Umsetzen der Schiffschen Base mit einer Carbonsäure in Gegenwart eines Lösungsmittels, dadurch gekennzeichnet, daß vor und/oder während der Umsetzung dem Reaktionsgemisch ebenfalls eine Menge an Wasser, welche zumindest der Menge an Schiff'scher Base äquivalent ist, zugegeben wird.

5. Verfahren nach irgendeinem der Ansprüche 1-4, wobei das Wasser zu Beginn der Umsetzung zugegeben wird.

6. Verfahren nach irgendeinem der Ansprüche 1-5, dadurch gekennzeichnet, daß das Reaktionsgemisch auf einer Temperatur zwischen 75 und 100°C gehalten wird.

7. Verfahren nach irgendeinem der Ansprüche 1-6, wobei das Aminosäureamid Phenylglycinamid ist.

8. Verfahren nach irgendeinem der Ansprüche 1-6, dadurch gekennzeichnet, daß das Aminosäureamid Alaninamid ist.

9. Verfahren nach irgendeinem der Ansprüche 1-6, dadurch gekennzeichnet, daß das Aminosäureamid Methioninamid ist.

10. Verfahren nach irgendeinem der Ansprüche 1-6, dadurch gekennzeichnet, daß das Aminosäureamid o-Chlorphenylglycinamid ist.

11. Verfahren nach irgendeinem der Ansprüche 1-10, dadurch gekennzeichnet, daß das erhaltene Salz aus dem racemisierten Aminosäureamid und der Carbonsäure anschließend gewonnen wird.

## Revendications

1. Procédé pour la racémisation d'un amide d'aminoacide optiquement actif consistant à faire réagir l'amide d'amino-acide avec un acide carboxylique en présence d'un solvant et d'un aldéhyde, caractérisé en ce que l'eau est ajoutée au mélange réactionnel et en ce que la quantité d'aldéhyde est de 0,5 à 4 équivalents par rapport à la quantité d'amine d'amino-acide.

2. Procédé selon la revendication 1, caractérisé en ce que la quantité d'eau est de 0,5 à 3 équivalents par rapport à l'amide.

3. Procédé selon les revendications 1 et 2, caractérisé en ce que la quantité d'aldéhyde est de 1 à 2 équivalents, par rapport à la quantité de l'amide d'amino-acide.

**4.** Procédé pour la racémisation d'une base de Schiff optiquement active d'un amide d'amino-acide consistant à faire réagir la base de Schiff avec un acide carboxylique en présence d'un solvant, caractérisé en ce que avant et/ou pendant la réaction, on ajoute également au mélange réactionnel une quantité d'eau qui est au moins équivalente à la quantité de la base de Schiff.

**5.** Procédé selon l'une quelconque des revendications 1 à 4, dans lequel on ajoute l'eau au début de la réaction

**6.** Procédé selon l'une quelconque des revendications 1 à 5, caractérisé en ce que l'on maintient le mélange réactionnel à une température comprise entre 75 et 100°C.

**7.** Procédé selon l'une quelconque des revendications 1 à 6, dans lequel l'amide d'amido-acide est l'amide de phénylglycine.

**8.** Procédé selon l'une quelconque des revendications 1 à 6, caractérisé en ce que l'amide d'amino-acide est l'amide d'alanine.

**9.** Procédé selon l'une quelconque des revendications 1 à 6, caractérisé en ce que l'amide d'amino-acide est l'amide métionine.

**10.** Procédé selon l'une quelconque des revendications 1 à 6, caractérisé en ce que l'amide d'amino-acide est l'amide o-chlorophénylglycine.

**11.** Procédé selon l'une quelconque des revendications 1 à 10, caractérisé en ce que l'on récupère le sel résultant de l'amide d'amino-acide racémisé et de l'acide carboxylique.